# EUROPEAN PATENT APPLICATION

(11) **EP 4 212 618 A1**
(43) Date of publication of application: **19.07.2023**
(21) Application number: 22382013.5
(22) Date of filing: 12.01.2022
(51) Int. Cl.: C12N 5/0783, C07K 14/705

(54) **METHOD OF PRODUCING VDELTA1+ T CELLS**

(71) Applicant: Consejo Superior de Investigaciones Científicas (CSIC), 28006 Madrid (ES); Onechain Immunotherapeutics SL, 08021 Barcelona (ES)
(72) Inventor: GARCÍA LEÓN, María Jesús, 28049 Madrid (ES); FUENTES VILLAREJO, Patricia, 28049 Madrid (ES); ALCAIN SÁNCHEZ, Juan, 28049 Madrid (ES); TORIBIO GARCÍA, María Luisa, 28049 Madrid (ES)
(74) Representative: Hoffmann Eitle

(57) **Abstract**

The present invention refers to a novel and efficient method for large-scale selective generation of γδ T cells, preferably human Vδ1+ γδ T cells, optimal for clinical application in adoptive immunotherapy of cancer. In this sense, considering that both human cord blood HPCs, currently elected as source of stem cells in the clinic, and human early thymic progenitors can efficiently generate *de novo* human γδ T cells in response to Notch signalling, and most efficiently in response to the Notch ligand Jag2, the method thus comprises inducing the differentiation of cord blood CD34+ hematopoietic progenitor cells (HPCs) and/or human CD34+ early thymic progenitors, by activating them with Jag2 Notch ligands.

## Description

### TECHNICAL FIELD

The present invention pertains to the medical field, in particular, the present invention refers to a novel and efficient method for large-scale selective generation of human Vδ1+ γδ T cells, optimal for clinical application in adoptive immunotherapy of cancer.

### BACKGROUND OF THE INVENTION

Barriers imposed by MHC (major histocompatibility complex) disparity often limit the use of adoptive T-cell therapies. Consequently, current clinical applications of T-cell products such as chimeric antigen receptor (CAR)-expressing T cells (CAR-T) rely on case-by-case autologous T-cell production. However, patient T cells are often functionally damaged due to the continuous administration of aggressive drug therapies. Also, the individualized custom-made autologous T-cell production process imposes constrictions on the wide application of T cells for particular tumour types, such as T-cell tumours. Therefore, universal allogeneic T cells are needed for the preparation of T-cell products that can serve as "off-the-shelf" ready-to-use therapeutic agents for large-scale clinical applications.

Recently, γδ T cells have emerged as an alternative to αβ T cells for cellular immunotherapy, as they are not constrained by MHC presentation of tumour-associated peptides and display limited allogeneic potential. Nonetheless, γδ T cells play an important role during viral infections and tumour progression, providing robust and durable antitumor responses (Vantourout and Hayday, Nat Rev Immunol., 2013*;* Silva-Santos et al., Nat. Rev. Immunol., 2015*).* In particular, Vδ1+ γδ T cells are very attractive candidates for adoptive cell therapy of cancer, as they are usually predominant (over Vδ2+) in tumour infiltrates, are less susceptible to activation-induced cell death, and can persist long-term as tumour-reactive lymphocytes (Siegers et al., Mol. Ther. 2014). However, Vδ1+ γδ T cells, which represent the prevalent Vδ1+ T cell subtype of γδ T cells at birth (Morita et al., J. Immunol. 1994), are poorly represented in the peripheral blood, and lack of suitable expansion/differentiation methods has precluded their therapeutic use. In this sense, the group of Bruno Silva-Santos has recently developed a two-step clinical-grade method, using TCR agonists and cytokines, for selective expansion of cytotoxic Vδ1 T cells isolated from the peripheral blood (Correia et al., Blood 2011*;* Almeida et al. Clin. Cancer Res. 2016). The cell product, named Delta One T (DOT) cells, showed therapeutic potential in preclinical models of chronic lymphocytic leukemia (CLL) providing the proof of principle for their clinical application in adoptive immunotherapy of cancer.

Still, the limited numbers of Vδ1+ T cells which can be isolated from peripheral blood stresses the need for developing complementary protocols for robust generation/expansion of cytotoxic Vδ1+ antitumoral T cells. More importantly, the unfocused and diverse T cell receptor (TCR) repertoire of neonatal Vδ1+ T cells becomes strongly restricted and focused on a few dominant clonotypes by adulthood (Davey et al., Nature Commun. 2016) due to clonal expansion in response to peripheral immune challenges such as CMV (Ravens et al., Nature Immunol. 2017). Clonal expansions of Vδ1+ cells lead to differentiation from a Vδ1 T cell naïve to a Vδ1T cell effector/memory phenotype characterized by CD27 downregulation (Davey et al., Trends Immunol. 2018). As human naive-derived effector T cells retain longer telomeres, are most capable of in vitro expansion and T-cell receptor transgene expression, and have been linked to greater efficacy in clinical trials, it has been postulated that naive cells resist terminal differentiation or "exhaustion", maintain high replicative potential, and therefore may be the superior subset for use in adoptive immunotherapy (Hinrichs et al., Blood 2011).

### BRIEF DESCRIPTION OF THE INVENTION

An object of the present invention, which was made to solve the problem above, refers to a novel and efficient method for large-scale selective generation of human Vδ1+ γδ T cells, preferably allogenic human Vδ1+ γδ T cells, optimal for clinical application in adoptive immunotherapy of cancer. In this sense, considering the findings of the present invention that both human umbilical cord blood CD34+ hematopoietic stem/progenitor cells (HPCs), currently elected as a source of hematopoietic stem cells in the clinic, and human CD34+ early thymic progenitors (ETPs) can efficiently generate *de novo* human Vδ1+ γδ T cells in response to Notch signalling, the method of the present invention thus comprises inducing the differentiation of human HPCs, preferably cord blood CD34+ HPCs, and/or human CD34+ ETPs, by activating them with the Jag2 Notch ligand, wherein said ligand is overexpressed on the surface of a bone marrow-derived stromal cell line. Preferably, the method comprises co-culturing for up to 9 weeks human HPCs, preferably cord blood CD34+ HPCs, or human CD34+ ETPs, onto Jag2-overexpressing stromal cells, preferably supplemented with Flt3+SCF+IL-7. Then, the Vδ1+ γδ T cells produced as described above in a Notch-dependent TCR-independent manner (STEP1), will be activated and expanded following any method known in the art to induce Vδ1+ γδ T cells' proliferation upon TCR activation, such as by using anti-CD3 mAbs and cytokines including IL-4, IFN-γ and IL-15 (Almeida et al., Clin. Cancer Res. 2016) (STEP2). It is important to note that, the method already described in the prior art (Almeida et al., Clin. Cancer Res. 2016*)* allows for an approx. 2000-fold expansion of peripheral blood Vδ1+ T cells, while the two-step method of the present invention may allow for an even greater Vδ1+ T-cell yield.

### BRIEF DESCRIPTION OF THE INVENTION

In one aspect, the present invention relates to the *in vitro* use of the Jag2 Notch ligand to generate a cell composition that comprises a higher amount of γδT cells than αβ T cells from a cell population comprising human HPCs, such as CD34+ cord blood HPCs, and/or CD34+ ETPs, wherein the Jag2 Notch ligand is expressed on the surface of a stromal cell line, and wherein said stromal cell line presents a statistically significant increased expression of the Jag2 Notch ligand as compared to a reference stromal cell, wherein said reference stromal cell is a stromal cell expressing basal levels of said ligand. Preferably, the stromal cell line is OP9 cell line.

In a further aspect, the present invention relates to an *in vitro* method to generate a cell composition that comprises a higher amount of γδ T cells than αβ T cells, preferably allogenic Notch-induced differentiated γδ T cells, from a cell population comprising human HPCs such as CD34+ cord blood HPCs, and/or human ETPs, the method comprising:
a. cultivating the cell population comprising the HPCs and/or ETPs in an adequate culture medium comprising a cell population of a stromal cell line, wherein said stromal cell line presents a statistically significant increased expression of the Jag2 Notch ligand as compared to a reference stromal cell, wherein said reference stromal cell is a stromal cell expressing basal levels of said ligand; and
*b.* maintaining the cells in culture for a duration of time sufficient to produce the γδ T cells, preferably the duration of time is between about 2 and about 15 weeks, and the said Notch ligand or agonist should be present or added in an adequate amount to said culture of cells at the time of the culturing and also throughout the culturing period.

Preferably, the human HPCs are derived from CD34+ cord blood HPCs, CD34+ bone marrow HPCs, CD34+ peripheral blood HPCs, or from CD34+ cells derived from pluripotent stem cells such as iPSCs. Preferably, the human hematopoietic progenitor cells are human CD34+ ETPs. In an embodiment, at least 30% of the γδ T cells are Vδ1+ γδ T cells.

The invention further provides a cell composition comprising a higher amount of γδ T cells than αβ T cells, obtained or obtainable according to any of the previous the aspects. Preferably, said cell composition is characterized in that at least 30% of the γδ T cells are Vδ1+ γδ T cells. Preferably, said cell composition is further characterized in that the population of Vδ1+ γδ T in turn comprises:
a. a first cell population characterized in that they express the immature surface cell marker CDla (CD1a+ Vδ1+ γδ T cells), and
b. a second cell population characterized in that they do not express the immature surface cell marker CDla (CD1a- Vδ1+ γδ T cells).

Preferably, the first cell population is characterized in that the cells do not express the surface cell markers CD25, CD27, NKp44, NKp30, and NKG2D and the second cell population is characterized in that the cells express at least one or at least a combination of two or more, preferably all, of the surface markers CD27, CD73, CD69, NKp44, NKp30, and NKG2D. Most preferably, the first cell population represents at least 90% of the total Vδ1+ γδ T cells comprised in the cell composition.

The present invention also provides a method of activating and inducing proliferation of the Vδ1+ γδ T cells obtained by any of the previous aspects, the method comprising:
a. cultivating a cell composition comprising the Vδ1+ γδ T cells obtained by the method of any of the previous aspects in an adequate culture medium in the presence of yδTCR agonists, preferably by regular addition (more preferably continuous addition) of said agonists, preferably soluble or immobilized, and in the presence of at least one cytokine such as those selected from the list consisting of IL-2, IL-4, IL-7, IL-9, IFN-y, IL-21, IL-15 and/or IL-1β; and
b. adding said yδTCR agonists and cytokines until at least 40% of the cells express natural cytotoxicity receptors, more preferably at least 50%, 60%, 70%, 75%, 80%, 85%, 95% of the cells express natural cytotoxicity receptors.

### BRIEF DESCRIPTION OF THE FIGURES

The following figures provide preferred embodiments for illustrating the description and should not be seen as limiting the scope of invention.

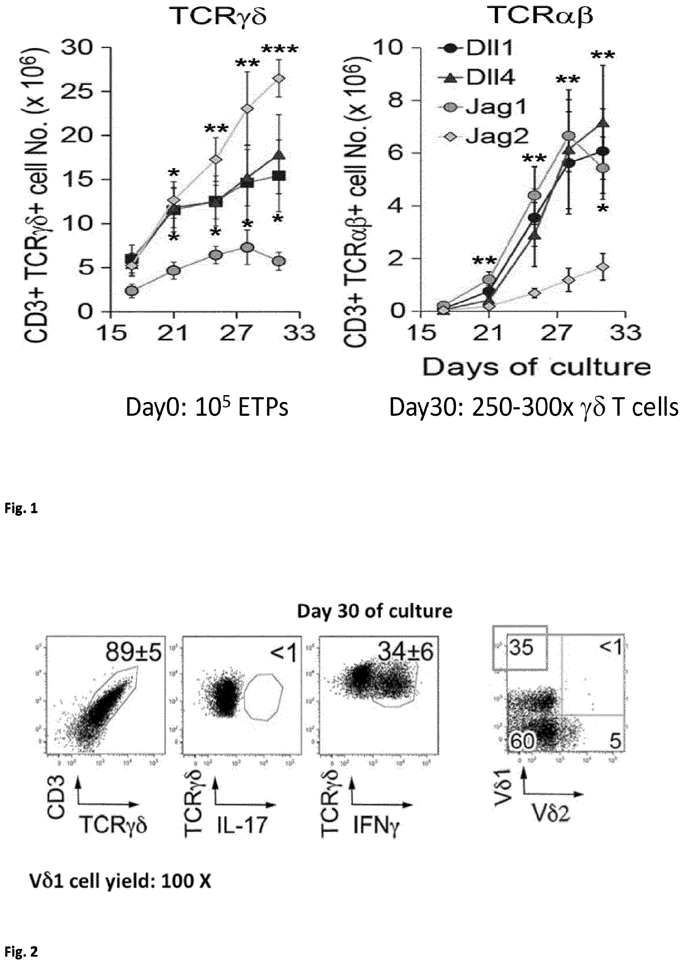

**Figure 1****.** Notch-induced γδ (and αβ) T-cell generation from CD34+ early human thymic progenitors (ETPs), wherein Jag2 is the most efficient ligand for γδ T cell generation. Jag2 induces a 250-300-fold γδ T-cell generation yield from CD34+ human ETPs by day30 of culture.
**Figure 2****.** Jag2-Notch signalling supports Vδ1+ γδ T-cell generation from ETPs. Jag2-induced Vδ1+ γδ T-cells generated from human CD34+ ETPs by day 30 of culture (100-fold yield) produce IFNγ but not IL-17.

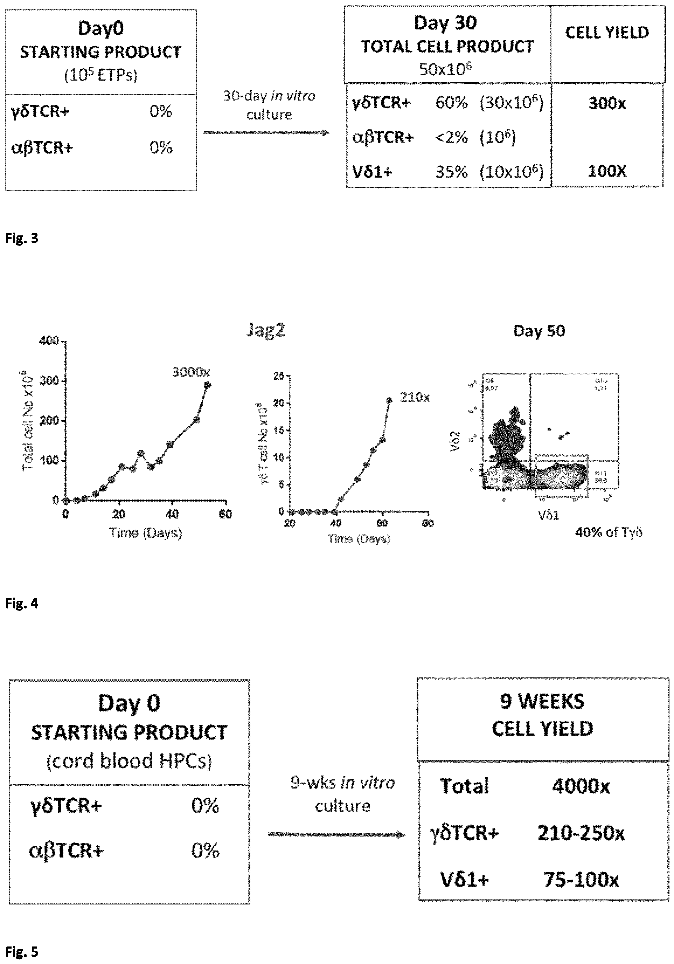

**Figure 3****.** Efficiency of Jag2-mediated *in vitro* Vδ1+ γδ T-cell production from human CD34+ ETPs.
**Figure 4****.** Jag2-Notch-induced Vδ1+ γδ T-cell generation from human CD34+ cord blood hematopoietic stem/progenitor cells (HPCs). Kinetics of total cell expansion and γδ T-cell generation induced by Jag2 from human CD34+ cord blood HPCs are shown. Dot plots show expression of Vδ1 on γδ T-cells generated *de novo* by day 50.
**Figure 5****.** Efficiency of total γδ and Vδ1+ γδ T-cell production from human CD34+ cord blood HPCs upon Jag2-Notch signalling in 9-weeks cultures (STEP1).

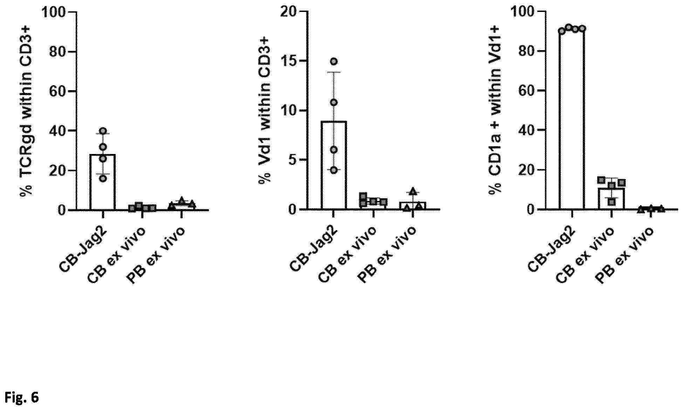

**Figure 6****.** Expression of activation, differentiation and cytotoxic markers by immature CD1a+ and_mature CD1a- Vδ1+ γδ naïve T cells generated from human CD34+ CB HPCs receiving human Jag2 signalling (CB-Jag2), or by Vδ1+ γδ T cells ex vivo-isolated from cord blood (CB ex vivo) or peripheral blood (PB ex vivo). Each dot represents one independent experiment or biological sample (n=4). One-way ANOVA, Tukey's multiple comparisons test.

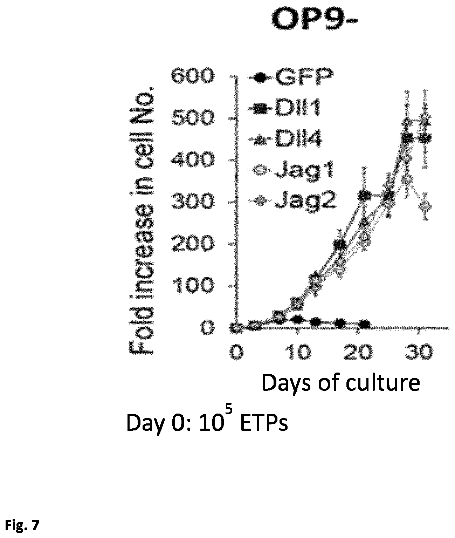

**Figure 7****.** Notch-induced γδ (and αβ) T-cell generation from CD34+ early human thymic progenitors (ETPs), wherein stromal cells lacking human Notch ligands (OP9-GFP) are unable to support ETP cell expansion/differentiation.

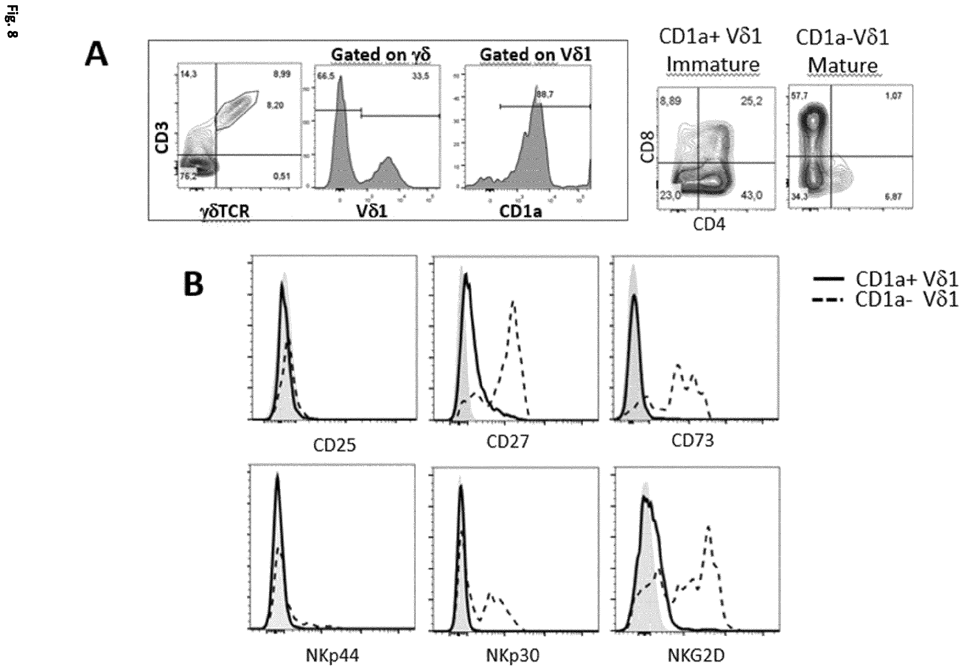

**Figure 8****.** Phenotype of Vδ1+ γδ T cells generated from human CD34+ CB HPCs receiving human Jag2 signaling. (A) Vδ1 + γδ T cells include a CD1a+ immature γδ T subset that displays either a CD4+, or a CD4+CD8+ double positive (DP), or a CD4-CD8- double negative (DN) phenotype, and a CD1a- mature γδ T cell subset of DN or CD8+ cells. (B) Mature CD1a- Vδ1+ γδ T cells are naïve CD27+ expressing the γδ T cell differentiation marker CD73 and distinct levels of NKp44, NKp30 and NKG2D cytotoxic NK receptors.

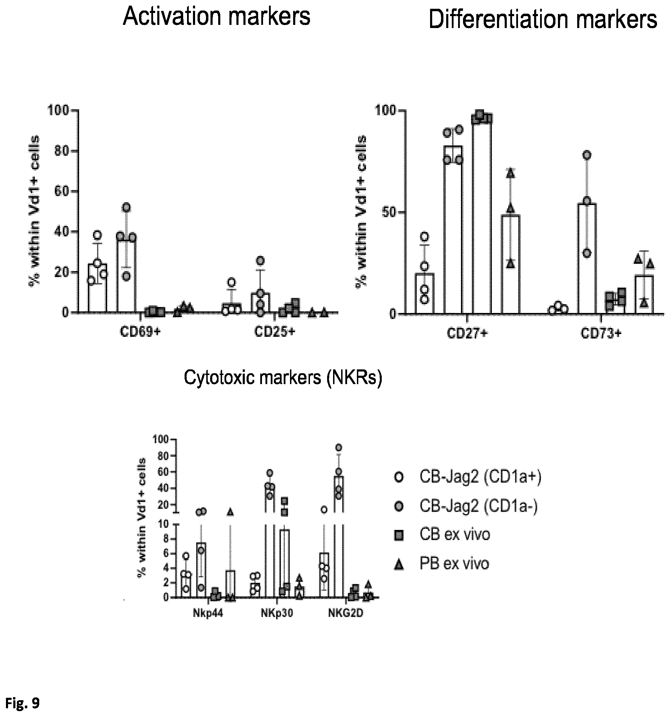

**Figure 9****.** Mature CD1a- Vδ1+ γδ naïve T cells generated from human CD34+ CB HPCs receiving human Jag2 signalling differ from Vδ1+ cells resident in PB in the expression of CD73 and cytotoxic NK receptors. Each dot represents one independent experiment or biological sample. One-way ANOVA, Tukey's multiple comparisons test.

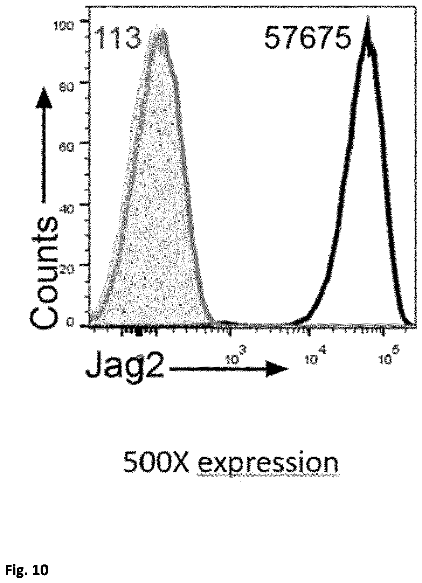

**Figure 10****.** Increased expression (overexpression) of Jag2 Notch ligands in transduced OP9 cells. Numbers indicate Mean Fluorescence Intensity of Jag2 expressed on the cell surface of OP9 stromal cells that were either non-transduced (left pick) or transduced with Jag2 (right pick). Cells were labelled with a PE-coupled mAb against human Jag2 and analysed by flow cytometry.

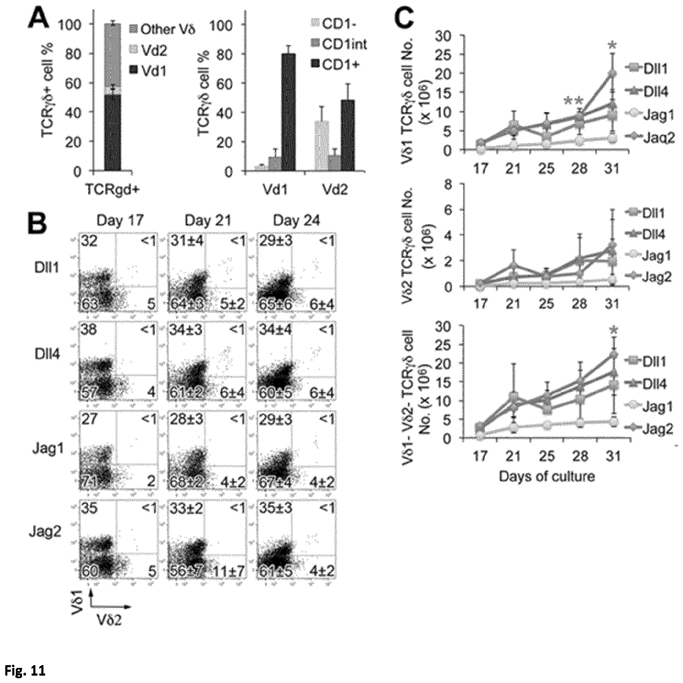

**Figure 11****.** Heterogeneity of δ5 subsets of γδ thymocytes developing in in vitro. (A) Bar graph represent mean +/- SEM frequencies of the indicated Vδ subpopulations of total γδ T cells (left) present in vivo in the human thymus; right: mean +/- SEM frequencies of CD1a+, CDla with low expression CD1a^{int} and CD1a-within the Vδ1 and Vδ2 γδ T cell subsets *in vivo* in the human thymus. (B) Flow cytometry kinetics analysis of Vδ1 and Vδ2 expressed on human ETP thymocytes co-cultured onto the indicated Notch ligand OP9 cell lines. Numbers in quadrants shown mean +/- SEM frequencies of the indicated cell subsets. (C) Kinetics of absolute Vδ1+, Vδ2+ and Vδ1-Vδ2- γδ T cell numbers generated from ETPs cultured as in B for 31 days. Data show mean +/- SEM cell numbers of 3 independent experiments.

### DESCRIPTION OF THE INVENTION

### Definitions

Unless defined otherwise, all technical and scientific terms used herein have the same meaning as is commonly understood by one of skill in the art to which this invention belongs at the time of filing. However, in the event of any latent ambiguity, definitions provided herein take precedent over any other definition. Further, unless otherwise required by context, singular terms shall include pluralities and plural terms shall include the singular forms as well.

In this disclosure, "comprises," "comprising," "containing" and "having" and the like can have the meaning ascribed to them in European and U.S. Patent law and can mean "includes," "including," and the like; "consisting essentially of" or "consists essentially" likewise has the meaning ascribed in European and U.S. Patent law and the term is open-ended, allowing for the presence of more than that which is recited so long as basic or novel characteristics of that which is recited is not changed by the presence of more than that which is recited, but excludes prior art embodiments.

The present invention is primarily directed to *de novo generation of* Notch-induced Vδ1+ γδ T cells differentiated from a cell population comprising human HPCs, such as CD34+ cord blood HPCs, and/or CD34+ ETPs. "De novo" is a Latin expression meaning "a new" or "from the beginning", that is, as used herein *de novo* shall be understood as the creation of a new Notch-induced differentiated Vδ1+ γδ T cells not based on previously existing Vδ1+ γδ T cells.

In the context of the present invention, "Notch ligands" are understood as proteins able to bind to surface Notch receptors, which provide cellular signals that mediate cell fate decisions, including activation and differentiation of hematopoietic progenitors (Artavanis-Tsakonas et al., Science 1999). The term as used herein therefore includes naturally occurring protein ligands such as Delta and Serrate/Jagged family ligands, as well as engineered Notch ligands and Notch agonists including antibodies to the Notch receptors, peptidomimetics and small molecules which have corresponding biological effects to the natural ligands. In some embodiments, the Notch ligand is the Jag2 Notch ligand. Preferred Notch ligands are selected from the list consisting of DLL1, DLL4, Jag1 or Jag2.

As used herein, "DLL1" is understood as a naturally occurring human homolog (Delta-like 1) of the Drosophila Notch Delta ligand. This term may further preferably include engineered Notch ligands and Notch receptor agonists with the biological effects of the natural Delta-like 1 ligand.

As used herein, "DLL4" is understood as a naturally occurring human homolog (Delta-like 4) of the Drosophila Notch Delta ligand. This term may further preferably include engineered Notch ligands and Notch receptor agonists with the biological effects of the natural Delta-like 4 ligands.

As used herein, "Jag1" is understood as a naturally occurring human homolog (Jagged 1) of the Drosophila Notch Serrate/Jagged ligand. This term may further preferably include engineered Notch ligands and Notch receptor agonists with the biological effects of the natural Jagged 1 ligand.

As used herein, "Jag2" is understood as a naturally occurring human homolog (Jagged 2) of the Drosophila Notch Serrate/Jagged ligand. This term may further preferably include engineered Notch ligands and Notch receptor agonists with the biological effects of the natural Jagged 2 ligand.

As used herein, "Vδ1 + γδ T cells" are understood as T cells expressing a TCR composed of a γ chain bound to a δ chain that expresses the Vδ1 variable region, and to CD3 components. Vδ1+ γδ T cells can be identified by phenotypic analyses using specific anti-Vδ1 antibodies or by sequencing of the Vδ region.

As used herein, "yδTCR agonists" are understood as antibodies, peptidomimetics, and small molecules that bind specifically to either the TCRγδ heterodimer, or to the Vδ1 domain of the TCRγδ, or to the TCR-associated CD3 components, mainly to the CD3ε component, inducing cell activation and proliferation.

As used herein, "human hematopoietic stem/progenitor cells (HPCs)" are understood as human CD34+ cells identified by phenotypic analyses with anti-CD34 antibodies and obtained *ex vivo* from human umbilical cord blood, placental blood, peripheral blood, bone marrow or foetal liver, or derived *in vitro* from pluripotent stem cells such as iPSCs (induced pluripotent stem cells).

As used herein, "CD34+ early thymic progenitors (ETPs)" are understood as human CD34+ cells identified by phenotypic analyses with anti-CD34 antibodies and obtained *ex vivo* from human foetal, neonatal or post-natal thymus.

In the context of the present invention "functional natural cytotoxicity receptors" (NCRs) shall be understood as surface receptors expressed by natural killer (NK) cells and also by human γδ T cells, almost exclusively by the Vδ1 + γδ T cell subset, following stimulation with strong TCR agonists or mitogens in the presence of IL-2 or IL-15 (Correia et al., Blood. 2011). NCR triggering plays a central role in cell activation, regulates cytotoxicity against primary leukemia cells and tumor cell-lines and enhances the expression of IFN-γ.

The term "overexpression" as used herein refers to a statistically significant increased expression of a Notch ligand in a cell as compared to the basal expression levels of said Notch ligand in a reference cell. The cell is preferably a mammalian cell, more preferably a stromal cell. An expression above basal levels includes pharmacological and artificial upregulation and overexpression of said Notch ligand. Overexpression of the Notch ligands or agonists thereof on a cell can be achieved by different means, such as by transfecting the cells with a plasmid encoding the gene for the Notch ligand operably linked to a suitable promoter for the expression of the Notch ligand in said cell, or by introducing the gene encoding for said Notch ligand into the cell's genome by using genetic engineering approaches, such as Crispr, integrative viral vectors, or homologous recombination methods. The term "cell of reference" or "reference cell" refers to an untreated control cell, i.e., a cell that has not been genetically modified nor artificially manipulated to induce the expression of said Notch ligand beyond the natural expression (i.e., the basal expression) that the cell may have. The reference cell is preferably a reference stromal cell. Thus, a reference stromal cell does not express the Notch ligand or expresses it at basal levels. A Notch ligand gene that is overexpressed on a cell or that has a statistically significant increased expression as compared to the basal expression levels of a cell can be detected by RNA expression techniques (Reverse transcription polymerase chain reaction, Fluorescent in situ hybridization, Northern blotting, etc.) or protein expression techniques (Western blotting, flow cytometry, etc.).

By "statistically significant increased expression of a Notch ligand" is referred herein as the determination by an analyst that the increase in the expression levels is not explainable by chance alone. Statistical hypothesis testing is the method by which the skilled person makes this determination. This test provides a p-value, which is the probability of observing results as extreme as those in the data, assuming the results are truly due to chance alone. A p-value of 0.1 or lower (preferably 0.05, 0.01, 0.001 or lower) is considered herein to be statistically significant. For example, the increase in the expression of the Notch ligand in a cell, preferably in a stromal cell, is statistically significant when a statistical test is performed to compare it to the basal expression levels of a reference cell, preferably a reference stromal cell, as defined above, and wherein the resulting p-value of said statistical test is of 0.1 or lower, preferably 0.05, 0.01, 0.001 or lower.

As used herein, the term "an adequate medium" shall be understood as any suitable mammalian cell culture medium Preferably, as any culture medium, preferably a serum-free culture medium (a-MEM) supplemented with 20% fetal calf serum and preferably L-glutamine (i.e. at a concentration of about 2mmol/l) and animal-free recombinant human (rh) cytokines such as IL-7 (200 IU/ml), Flt3L (100 IU/ml) and SCF (100IU/ml) for STEP1 of the method of the present invention; or serum-free culture medium (OpTimizer-CTS), optionally supplemented with autologous plasma (i.e. 5% autologous plasma) or human AB serum and preferably L-glutamine and animal-free recombinant human (rh) cytokines such as rh IL-4 (preferably at a concentration of about 100 ng/ml), IFN-γ (preferably at a concentration of about 70 ng/ml), IL-21 (preferably at a concentration of about 7 ng/ml), and IL-1β (preferably at a concentration of about 15 ng/ml) for STEP2 of the method of the present invention. It is noted that numerous basal culture media suitable for use in the proliferation of γδ T cells are available, in particular complete media, such as AIM-V, Iscoves medium and RPMI-1640 (Life Technologies). The medium may be supplemented with other media factors, such as serum, serum proteins and selective agents, such as antibiotics. For example, in some embodiments, RPMI-1640 medium containing 2 mM glutamine, 10% FBS, 10 mM HEPES, pH 7.2, 1 % penicillin-streptomycin, sodium pyruvate (1 mM; Life Technologies), non-essential amino acids (e.g. 100µM Gly, Ala, Asn, Asp, Glu, Pro and Ser; 1 X MEM non-essential amino acids Life Technologies), and 10 µl/L β-mercaptoethanol. The basal medium may be supplemented with IL-2 and/or IL-15 at standard concentrations which may readily be determined by the skilled person by routine experimentation.

By "treating", "to treat" or "treatment" is meant, without limitation, restraining, limiting, reducing, stabilizing, or slowing the growth of a disease.

By "medicament" or "medicinal product" is meant any pharmaceutical or veterinary composition (also referred to as medicine, medication, or simply drug) used to cure, treat or prevent disease in animals, including humans, as widely accepted.

By "pharmaceutical composition" is meant an active substance or combination of active substances intended to prepare a final medicinal product for prevention and/or therapeutic use.

By "Pharmaceutically acceptable" is meant a material that is not biologically or otherwise undesirable, i.e., the material can be administered to a subject along with the compositions of the invention without causing any undesirable biological effects or interacting in a deleterious manner with any of the components of such compositions. As used herein, the terms "pharmaceutically acceptable carrier" and "pharmaceutically acceptable vehicle" are interchangeable and refer to a vehicle for containing the active substances of a pharmaceutical composition that can be administered to a subject and/or the environment without adverse effects. Suitable pharmaceutically acceptable carriers include, but are not limited to, sterile water, purified water, saline, glucose, dextrose, or buffered solutions. Carriers may include auxiliary agents including, but not limited to, diluents, stabilizers, preservatives, wetting agents, dispersant agents, emulsifying agents, pH buffering agents (for example phosphate buffer), viscosity additives, and the like.

As used herein "autologous" is understood as referring to a cell preparation where the donor and the recipient are the same individual. As used herein "allogeneic" is understood as referring to a cell preparation where the donor and the recipient are not the same individual.

The term "isolated" indicates that the cell or cell population to which it refers is not within its natural environment. The cell or cell population has been substantially separated from surrounding tissue.

The marker profile of the cell composition product referred to in the present invention can be further defined by the presence and/or absence of additional markers, or by a specific profile of a combination of present and absent markers. In each case, the specific combination of markers may be present as a particular profile within a cell population and/or a particular profile of markers on individual cells within the population.

The term "marker" as used herein encompasses any biological molecule whose presence, concentration, activity, or phosphorylation state may be detected and used to identify the phenotype of a cell.

Then, cells of the invention are positive for certain phenotypic markers and negative for others. By "positive", it is meant that a marker is expressed within a cell. In order to be considered as being expressed, a marker must be present at a detectable level. By "detectable level" is meant that the marker can be detected using one of the standard laboratory methodologies such as PCR, blotting or flow cytometry analysis as described.

The term "expressed" is used to describe the presence of a marker on the surface of or within a cell. In order to be considered as being expressed, a marker must be present at a detectable level. By "detectable level" is meant that the marker can be detected using one of the standard laboratory methodologies such as PCR, blotting, immunofluorescence, ELISA or FACS analysis. "Expressed" may refer to, but is not limited to, the detectable presence of a protein, phosphorylation state of a protein or an mRNA encoding a protein. A gene is considered to be expressed by a cell of the invention or a cell of the population of the invention if expression can be reasonably detected after 25 PCR cycles, preferably after 30 PCR cycles, which corresponds to an expression level in the cell of at least about 75-100 copies per cell. The terms "express" and "expression" have corresponding meanings. At an expression level below this threshold, a marker is considered not to be expressed.

The term "cell population" refers to a group of cells. A cell population is heterogeneous when it comprises different groups of cells, wherein each group is differentiated from others by the presence of one or more distinguishing characteristics, such as the expression or not expression of specific markers or the presence of a different function.

The term "stromal cell" refers to bone marrow-derived stromal cell lines.

### Description

Hereinafter, favourable embodiments of the present invention will be described specifically with reference to the attached figures.

The thymus is the main organ for the generation *de novo* of both the major αβ and the minor γδ T cells subsets from HPCs. Vδ1+ γδ T cells represent the predominant γδ T-cell subset in the post-natal thymus, but the minor γδ T-cell subset in the peripheral blood. Generation of both αβ and γδ T cells in the thymus is dependent on Notch signalling being DII4 indispensable for *in vivo* T-cell commitment and development *(*Koch et al., J. Exp. Med. 2008*;* Hozumi et al., J. Exp. Med. 2008*).* In the present invention, we show that strong and maintained Notch signalling specifically provided by the overexpression of human Notch ligand Jag2 favors γδ over αβ T-cell generation, while overexpression of Notch signalling using any of the other Notch ligands (Jag1, DII4 and DII1) favors αβ over γδ T-cell generation (see Fig. 1). Based on these grounds and based on the knowledge of the authors of the present invention on *in vitro* methods of production *de novo* of human T cells from human thymic progenitors such as CD34+ ETPs, a culture method was developed that supports the preferential generation of human γδ T cells (an among them, Vδ1+ γδ T cells) from CD34+ hematopoietic progenitors obtained from any biological sample, such as from intrathymic and from cord blood hematopoietic progenitors. The method comprises coculturing said CD34+ cells onto the murine OP9 stromal cell line transduced with human Jag2. First, we found that ETPs that receive strong Notch signals from the four ligands (i.e. DII1, DII4, Jag1, Jag2) provided by their overexpression in OP9 cells expressed in the human thymus (García-León et al. Development 2018) can differentiate into both αβ and γδ T cells, while ETPs cultured onto non-transduced OP9 cells are unable to survive and/or differentiate *in vitro* and disappear from the cultures (see Figure 7). However, we also found that human Jag2 signaling is the only Notch ligand that selectively induces a differentiation pattern of ETPs distinct from that observed in the presence of other Notch ligands, with a very poor αβ T cell production and a preferential differentiation into γδ T cells (enriched up to 300-fold by day 30) (see Figure 1). As found *in vivo* in the post-natal human thymus (Figure 11), γδ T cells differentiated *in vitro* from ETPs in response to human Jag2 signalling overexpressed on OP9 cells showed a predominant expression of Vδ1 and Vδ1+ cells predominantly expand along culture in response to Jag2 (Figure 11). About 35% of γδ T cells were Vδ1+ (Figure 2), indicating that overexpression of Jag2 in OP9 cells leads to a 100-fold Vδ1+ T cell yield after 4 weeks of culture (Figure 3).

In addition, and remarkably, as shown in Figure 4, we herein demonstrate that human Jag2-Notch signalling also supports γδ T-cell generation *in vitro* from human cord blood CD34+ HPCs, and with similar efficiencies than from ETPs. In fact, production of total γδ T cells from cord blood HPCs was 210-250-fold enriched in OP9-Jag2 cultures after 9 weeks, with up to 40% of Vδ1+ γδ T cells (Figure 4). Therefore, we further propose to use cord blood as an optimal source of CD34+ progenitors for generating high numbers (up to about a 100-fold yield) of human Vδ1+ T cells (Figure 5), which can subsequently be expanded for adoptive cell therapy. Phenotypic analyses of the cells indicated that the Vδ1+ T cell population obtained is a heterogeneous cell population that includes non-activated CD25- naive γδ T cells, which, as found *in vivo* in the post-natal human thymus (Figure 11), comprises a major subset of immature CD1a+ cells that display either a CD4+, a double positive (DP) CD4+CD8+ or a CD4-CD8- double negative (DN) phenotype, and a minor population of mature CD1a- γδ T cells mostly composed of DN or CD8+ cells. Notably, mature CD1a- Vδ1+ γδ T cells display a naive CD27+ phenotype and express the γδ T cell differentiation marker CD73 and distinct levels of several cytotoxic NK receptors (see Figure 8), and are thus similar to naïve Vδ1+ fetal cells resident in cord blood, but distinct from adult peripheral blood Vδ1+ T cells (see Figure 9). Proportions and numbers of naïve Vδ1+ γδ T cells generated *de novo* from CD34+ HPCs isolated from a single CB unit and receiving human Jag2 signaling are significantly higher than those obtained *ex vivo* from single CB or peripheral blood units (Figure 9).

Hence, an object of the present invention refers to a novel and efficient method for large-scale selective generation of human γδ T cells, more preferably allogenic human Vδ1+ γδ T cells, optimal for clinical application in adoptive immunotherapy of cancer. In this sense, considering that both human cord blood HPCs, currently elected as a preferred source of stem cells in the clinic, and human ETPs can efficiently generate *de novo* human γδ T cells in response to Notch signalling, the method thus comprises inducing the differentiation of any biological sources comprising hematopoietic stem/progenitor cells, such as cord blood CD34+ HPCs, and/or human thymic ETPs through Notch activation mediated by a Notch ligand, preferably DLL1, DLL4, Jag1 or Jag2 Notch ligands, more preferably Jag2. In particular, the method comprises co-culturing, preferably for up to 15 weeks, preferably for up to 10, 9, 8, 7 6, 5 or up to 4 weeks, HPCs, preferably human CD34+ HPC cells, or human ETPs onto cells overexpressing Notch ligands, preferably human Jag2-overexpressing stromal cells, preferably supplemented with Flt3, SCF and IL-7. Then, the generated γδ T cells, which include a population of Vδ1+ γδ T cells, obtained as described above in a TCR-independent Notch-dependent manner (called herein STEP1), will be expanded following any method known in the art to activate and induce proliferation of said Vδ1+ γδ T cells, such as by using anti-CD3 mAbs and cytokines including IL-4, IFNγ and IL-15 (Almeida et al., Clin. Cancer Res. 2016*)* (called herein STEP2).

Therefore, a **first aspect** of the invention refers to the *in vitro* use of Notch ligands, preferably Jag2 Notch ligand, to generate a cell composition that comprises a higher amount of γδ T cells than αβ T cells from a cell population comprising human HPCs, such as CD34+ cord blood HPCs, and/or human ETPs. Preferably, the Notch ligand, preferably Jag2, is expressed on a cell, preferably a stromal cell, wherein said cell presents a statistically significant increased expression of said Notch ligand as compared to the basal expression levels of said ligand in a reference cell, preferably a reference stromal cell. Thus, provided herein is a Notch ligand, preferably Jag2, that is expressed above basal levels on a cell, preferably on its surface, and that is used to generate a cell composition that comprises a higher amount of γδ T cells than αβ T cells from a cell population comprising HPCs such as CD34+ cord blood HPCs, and/or CD34+ ETPs. Preferably, the cell where the Notch ligand is overexpressed is a stromal cell line, most preferably is OP9 cells. The terms "reference cell" and "statistically significant increased expression" have been defined above and apply throughout the whole patent description.

The degree of Notch ligand, preferably Jag2, overexpression in a cell, preferably a stromal cell, may be of about 100, 200, 300, 400, 500, 600, 700, 800, 900, 1000, 10000, 100.000-fold increased expression as compared to the Notch ligand basal expression levels in a reference cell, preferably a reference stromal cell. Figure 10 shows an overexpression of 500-fold in a Jag2-transduced stromal cell in comparison to a reference (non-transduced) stromal cell. Expression of the Notch Ligand, preferably Jag2, above basal levels (i.e., overexpression) may be achieved by any method known to those skilled in the art. By way of example, expression above basal levels may be induced by modulating the regulation of native genomic Notch Ligand. This may be done by increasing transcription and/or translation of the Notch Ligand, and/or by introducing heterologous regulatory sequences into or adjacent the native regulatory region of the Notch Ligand, and/or by replacing the native regulatory region of the Notch Ligand with such heterologous regulatory sequences, e.g. by homologous recombination, and/or by disrupting or downregulating molecules that negatively regulate, block or downregulate transcription, translation or the function of said Notch Ligand.

Transcription of the Notch Ligand above basal levels may be increased by providing the cell, preferably the stromal cell, with increased levels of a transcriptional activator, e.g. by contacting the cell with such an activator or by transformation of the cell with nucleic acid encoding the activator. Alternatively, transcription may be increased by transforming the cell with antisense nucleic acid to a transcriptional inhibitor of the Notch Ligand.

As an alternative or addition to increasing transcription and/or translation of endogenous Notch Ligand, expression of the Notch Ligand above basal levels may be caused by introduction of one or more extra copies of the Notch Ligand into the cell, preferably stromal cell, for instance by transfecting or transducing the cell with a nucleic acid encoding for the Notch Ligand. The transformed Notch Ligand may be contained on an extra-genomic vector or it may be incorporated, preferably stably, into the genome. It may be operably-linked to a promotor which drives its expression above basal levels in the cell. "Operably linked" means joined as part of the same nucleic acid molecule, suitably positioned and oriented for transcription to be initiated from the promoter.

Methods of introducing genes into cells are well known to those skilled in the art. Vectors may be used to introduce the Notch Ligand into cells, whether or not the Notch Ligand remains on the vector or is incorporated into the genome. Suitable vectors can be chosen or constructed, containing appropriate regulatory sequences, including promoter sequences, terminator fragments, polyadenylation sequences, enhancer sequences. Vectors may contain marker genes and other sequences as appropriate. The regulatory sequences may drive expression of Notch Ligand within the cell. For example, the vector may be an extra-genomic expression vector, or the regulatory sequences may be incorporated into the genome with the Notch Ligand. Vectors may be plasmids or viral.

The nucleic acid comprising the coding sequence of the Notch Ligand may be integrated into the genome of the cell, preferably stromal cell. Integration may be promoted by including in the transduced or transfected nucleic acid sequences which promote recombination with the genome, in accordance with standard techniques. The integrated nucleic acid may include regulatory sequences able to drive expression of then Notch Ligand above basal levels. The nucleic acid may include sequences which direct its integration to a site in the genome where Notch Ligand coding sequence will fall under the control of regulatory elements able to drive and/or control its expression within the cell. The integrated nucleic acid may be derived from a vector used to transduce or transfect the Notch Ligand nucleic acid into the cell.

The introduction of nucleic acid comprising the Notch Ligand, whether that nucleic acid is linear, branched or circular, may be generally referred to without limitation as "transfection" or "transduction". It may employ any available technique. Suitable techniques may include calcium phosphate transfection, DEAE-Dextran, electroporation, mechanical techniques such as microinjection, direct DNA uptake, receptor mediated DNA transfer, transduction using retrovirus or other virus and liposome-mediated transfection. When introducing a chosen gene construct into a cell, certain considerations must be taken into account, well known to those skilled in the art.

Suitable vectors and techniques for in vivo transfection or transduction of cells, preferably stromal cells, with the Notch Ligand to provide a cell, preferably a stromal cell, overexpressing said Notch ligand are well known to those skilled in the art. Suitable vectors include adenovirus, papovavirus, vaccinia virus, herpes virus and retroviruses. Disabled virus vectors may be produced in helper cell lines in which genes required for production of infectious viral particles are expressed. Suitable helper cell lines are well known to those skilled in the art.

In a preferred embodiment, the Notch ligand overexpression, i.e., the statistically significant increase in the expression levels of a Notch ligand in a cell, preferably a stromal cell, is achieved by transducing or transfecting said cell with a nucleic acid comprising the gene encoding for said Notch ligand, preferably human Jag2 Notch ligand, operably linked to a strong expression promoter, such as cytomegalovirus (CMV), SV40, elongation factor (EF)-1 promoters. Thus, in an embodiment of the first aspect of the invention, the HPCs such as CD34+ cord blood HPCs, and/or CD34+ ETPs, are cultured in a medium that includes a Notch ligand, preferably human Jag2, overexpressed in a cell line, preferably in a stromal cell line, preferably in OP9 cells, wherein said cell line is transduced or transfected with a nucleic acid comprising the gene for said Notch ligand which drives the overexpression of said Notch ligand in the cell.

Useful sources comprising CD34+ HPCs useful in the present invention are bone marrow and/or peripheral blood comprising HPCs, preferably HPCs mobilized from bone marrow. Other sources comprising HPCs include placental blood and foetal liver as well as CD34+ cells derived from pluripotent stem cells such as iPSCs (induced pluripotent stem cells), which may also be suitable to carry out the present invention. Other sources comprising HPCs include placental blood, foetal liver or CD34+ cells derived from pluripotent stem cells such as iPSCs (induced pluripotent stem cells), which may also be suitable to carry out the present invention. Preferably, the cell population comprising human HPCs such as CD34+ cord blood HPCs, and/or CD34+ ETPs, from which the Notch-induced differentiated γδ T cells, preferably Vδ1+ γδ T cells are derived, is a substantially pure or homogenous population. In the context of the present invention, a substantially pure population is a population wherein the HPCs such as CD34+ cord blood HPCs, and/or CD34+ ETPs, may be substantially isolated cells. In one embodiment, the HPCs such as CD34+ cord blood HPCs, represent at least about 75%, at least about 80%, at least about 85%, at least about 90%, at least about 95%, at least about 98%, at least about 99% or 100% of the cells in the composition. In another embodiment, the human CD34+ ETPs represent at least about 75%, at least about 80%, at least about 85%, at least about 90%, at least about 95%, at least about 98%, at least about 99% or 100% of the cells in the composition.

In other embodiments of the first aspect of the invention, the HPCs such as CD34+ cord blood HPCs, and/or CD34+ ETPs, are cultured in a medium that includes a population of cells, preferably stromal cells, overexpressing an engineered Notch ligand, preferably Jag2, or Notch agonists including antibodies to the Notch receptor, peptidomimetics, and small molecules, which have the biological effects of the natural ligands, or are cultured with such compounds immobilized on a substrate (i.e. on a plastic surface or on microbeads). Preferably, the HPCs such as CD34+ cord blood HPCs, and/or CD34+ ETPs, may be further cultured in a medium that includes a Notch ligand, preferably Jag2, or Notch agonists including antibodies to the Notch receptor, peptidomimetics, and small molecules, which have the biological effects of the natural ligands, immobilized on a cell line. Preferably, the Notch ligand is a human Notch ligand, preferably human Jag2. In some aspects of the invention, the Notch ligand or the Notch agonist can be immobilized on a solid substrate suspended in the medium, which facilitates interaction of the HPCs such as CD34+ cord blood HPCs and/or CD34+ ETPs, and the Notch ligand or Notch agonist. The method further includes maintaining the cells in culture for a duration of time sufficient to produce the Vδ1+ γδ T cells. In some embodiments, the duration of time is between about 2 and about 15 weeks, preferably between about 2 and 9 weeks.

Alternatively, the first aspect of the invention also refers to an *in vitro* method to generate a cell composition that comprises a higher amount of γδ T cells than αβ T cells from a cell population comprising human HPCs such as CD34+ cord blood HPCS, and/or from CD34+ ETPs. Further sources comprising HPCs useful in the present method are bone marrow or peripheral blood, preferably peripheral blood comprising HPCs mobilized from bone marrow. Other sources comprising HPCs include placental blood, foetal liver or CD34+ cells derived from pluripotent stem cells such as iPSCs, which may also be suitable to carry out the present method. Preferably, the method comprises cultivating the cell population comprising human HPCs such as CD34+ cord blood HPCS, and/or CD34+ ETPs in an adequate culture medium, preferably a medium that comprises a Notch ligand (preferably DLL1, DLL4, Jag1 or Jag2 Notch ligands, more preferably Jag2) or Notch receptor agonists immobilized on a substrate or overexpressed in a cell line. In some aspects of the invention, the Notch ligand or the Notch agonist can be immobilized on a solid substrate suspended in the medium, which facilitates interaction of the HPCs such as CD34+ cord blood HPCS, and/or CD34+ ETPs, and the Notch ligand or Notch agonist. In some other embodiments, the Notch ligand, preferably Jag2, is overexpressed on a stromal cell line. The method further includes maintaining the cells in culture for a duration of time sufficient to produce the composition that is enriched in γδ T cells, preferably Vδ1+ γδ T cells. In some embodiments, the duration of time is between about 2 and about 15 weeks, preferably between about 2 and 9 weeks. The said Notch ligand or Notch agonist may be present or added to said culture of cells at the time of the culturing and also throughout the culturing period. In a preferred embodiment, the Notch ligand, preferably Jag2, most preferably human Jag2, is overexpressed in a cell line, preferably stromal cells, preferably on their surface, wherein said cells are co-cultured to interact with the cell population comprising human HPCs such as CD34+ cord blood HPCS, and/or from CD34+ ETPs. The term "overexpression" has been explained above and applies herein.

In view of the above, the first aspect of the present invention provides the use of an overexpressed Notch ligand, preferably Jag2, and methods to generate a cell composition that comprises a higher amount of γδ T cells than αβ T cells, and that is obtained from a cell population comprising human HPCs such as CD34+ cord blood HPCS, and/or from CD34+ ETP. As shown in the examples, particularly in Example 1 and Figs. 1-3, the human Jag2 Notch ligand is the only ligand that, when overexpressed on a stromal cell line, selectively induces a differentiation pattern where γδ T cells are produced in higher proportion than αβ T cells. Thus, in an embodiment of the first aspect that can be applied to both the use and the methods as defined above, the overexpressed Jag2 Notch ligand is used herein to favor the generation of γδ over αβ T cells from a cell population comprising human HPCs, such as CD34+ cord blood HPCs, and/or CD34+ ETPs. Preferably, the cell composition that is enriched in γδ T cells is a population of T cells comprising both γδ T cells and αβ T cells, where the γδ T cells represent at least 80-95%, preferably 90-95%, of the total T cells after at least 30 days of culture according to the uses and methods of the first aspect. In an embodiment, the αβ T cells represent less than 15%, preferably between 5 and 10%, of the total T cells after at least 30 days of culture according to the use and methods of the first aspect. Preferably, at least 30%, preferably between 30-40% of the γδ T cells are Vδ1+ γδ T cells. Preferably, the Vδ1+ γδ T cells are allogenic Notch-induced differentiated Vδ1+ γδ T cells. A more detailed description of the γδ T cells obtained or obtainable according to the uses and method of the first aspect are provided in the second aspect of the invention.

A **second aspect** of the invention refers to the cell composition that comprises a higher amount of γδ T cells than αβ T cells obtained or obtainable from the use and method of the first aspect. Preferably, at least 80-95%, preferably 90-95%, of the total number of T cells of the cell composition are γδ T cells after at least 30 days of culture according to the use and methods of the first aspect. In an embodiment, less than 15%, preferably between 5 and 10% of the total number of T cells comprised in the cell composition are αβ T cells after at least 30 days of culture according to the use and methods of the first aspect.

In an embodiment of the second aspect, the γδ T cells comprised in the cell composition are in turn a heterogeneous cell population, wherein:
i) at least 30%, preferably between 30-40% of the total number of γδ T cells are Vδ1+ γδ T cells,
ii) at least 4%, preferably between 4-7% of the total number of γδ T cells are Vδ2+ γδ T, and
iii) about 50% of the total number of γδ T cells are neither Vδ2+ nor Vδ1+ cells.

Moreover, in a preferred embodiment, the Vδ1+ γδ T cells, preferably the Vδ1+ γδ T cells of i), are also a heterogeneous cell population that in turn comprises the following cell populations:
a) a first cell population characterized in that they express the immature surface cell marker CDla (named from herein after CD1a+ Vδ1+ γδ T cells), and
b) a second cell population characterized in that they do not express the immature surface cell marker CDla (named from herein after CD1a- Vδ1+ γδ T cells).

The heterogeneous Vδ1+ γδ T cell population comprising a) and b) is shown in the Examples, particularly in Figs 8 and 9.

In an embodiment, the first population of T cells (CD1a+ Vδ1+ γδ T cells) represents the majority of the Vδ1+ γδ T cells comprised in the heterogeneous Vδ1+ γδ T cell population. Preferably, at least 80%, 85%, 90%, 91%, 92%, 93%, 94%, 95%, 96%, 97%, 98%, or 99%, preferably between 85-95%, of the total number of Vδ1+ γδ T cells are CD1a+ Vδ1+ γδ T cells (first population). Thus, the population of Vδ1+ γδ T cells is enriched in CD1a+ Vδ1+ γδ T cells. Preferably, between 8-12% of the total number of Vδ1+ γδ T cells are CD1a- Vδ1+ γδ T cells (second population).

In a preferred embodiment, the cell composition obtainable or obtained from the methods and uses of the first aspect is a heterogeneous cell population enriched in γδ T cells since at least 80-95%, preferably 90-95% of the total number of T cells are γδ T cells after at least 30 days of culture, and wherein:
i) at least 30%, preferably between 30-40% of the total number of γδ T cells are Vδ1+ γδ T cells,
ii) at least 4%, preferably between 4-7% of the total number of γδ T cells are Vδ2+ γδ T, and
iii) about 50% of the total number of γδ T cells are neither Vδ2+ nor Vδ1+ cells,
and wherein the population of i) is enriched in CD1a+ Vδ1+ γδ T cells since 85-95% of the total Vδ1+ γδ T cells are CD1a+ Vδ1+ γδ T cells and between 8-12% of the total Vδ1+ γδ T cells are CD1a- Vδ1+ γδ T cells.

Preferably, the Vδ1+ γδ T cells produce IFNγ but not IL-17, see Figure 2.

Methods of measurement of each of the cell populations and their proportions mentioned above are known in the art and established in the description of the invention. In particular, methods of measurement of each of type of T cells are conducted using total and differential cell counts with an automated cell counter. Cell percentages and CDla expressing cell percentages can be determined using a flow cytometer.

In an embodiment of the second aspect, the first population of cells (CD1a+ Vδ1+ γδ T cells) are further characterized in that they do not express at least one, preferably all, of the following surface markers CD25, CD27, NKp44, NKp30, and NKG2D. In an embodiment, the cells of the first population may or may not express CD4, CD8, or both, i.e., they can be CD4+CD8-, CD4-CD8+, CD4+CD8+ or CD4-CD8-, see Figs. 8 and 9.

In an embodiment of the second aspect, the second population of cells (CD1a- Vδ1+ γδ T cells) are further characterized in that they express at least one or at least a combination or two or more, preferably all, of the following surface markers CD27, CD73, CD69, NKp44, NKp30, and NKG2D, see Figs. 8 and 9.

Preferably, the cell composition according to the second aspect comprises human allogenic cytotoxic Vδ1+ γδ cells obtained or obtainable according to the first aspect of the invention.

A **third aspect** of the invention refers to a cell composition comprising the first population of Vδ1+ γδ T cells as defined in the second aspect. Thus, the cell composition according to the third aspect comprises Vδ1+ γδ T cells characterized in that they express at least the CDla surface marker and, preferably, they do not express at least one, preferably all, of the following surface markers CD25, CD27, NKp44, NKp30, and NKG2D. A **fourth** aspect of the invention refers to a cell composition comprising the second population of Vδ1+ γδ T cells as defined in the second aspect. Thus, the cell composition according to the fourth aspect comprises Vδ1+ γδ T cells characterized in that they do not express the CDla surface marker and, preferably, they express at least one or at least a combination of two or more, preferably all, of the following surface markers CD27, CD73, CD69, NKp44, NKp30, and NKG2D.

In a preferred embodiment, the cell population of the third and fourth aspect is a substantially pure or homogenous population. In the context of the present invention, a substantially pure population is a population wherein the cells may be substantially isolated cells. In one embodiment, the cell or cell population represent at least about 75%, at least about 80%, at least about 85%, at least about 90%, at least about 95%, at least about 98%, at least about 99% or 100% of the cells in the composition.

A **fifth aspect** of the invention refers to a heterogeneous cell population of autologous or allogeneic, Vδ1+ γδ T cells, wherein said heterogeneous cell population comprises a first and a second subpopulations of Vδ1+γδ T cells, wherein the first subpopulation comprises cells expressing the immature surface cell marker CDla (CD1a+ Vδ1+ γδ T cells), and wherein the second subpopulation comprises cells that do not express the immature surface cell marker CDla (CD1a- Vδ1+ γδ T cells). In an embodiment, the first subpopulation of cells (CD1a+ Vδ1+γδ T cells) represents the majority of the cells comprised in the heterogeneous cell population. Preferably, the first subpopulation of cells represents at least 60%, 70%, 80%, 90%, 95%, 96%, 97%, 98%, or 99% of the total Vδ1+γδ T cells comprised in the heterogeneous cell composition. Methods of measurement of each of the cell subpopulations and their proportions mentioned are known in the art and established in the description of the invention. In particular, methods of measurement of each of the Vδ1+γδ T cells are conducted using total and differential cell counts with an automated cell counter. Cell percentages and CDla expressing cell percentages can be determined using a flow cytometer.

In an embodiment of the fifth aspect, the cells of the first subpopulation (CD1a+ Vδ1+γδ T cells) are further characterized in that they do not express at least one, preferably all, of the following surface markers CD25, CD27, NKp44, NKp30, and NKG2D. In an embodiment, the cells of the first subpopulation may or may not express CD4, CD8, or both, i.e., they can be CD4+CD8-, CD4-CD8+, CD4+CD8+ or CD4-CD8-, see Fig. 8.

In an embodiment of the fifth aspect, the cells of the second subpopulation (CD1a- Vδ1+ γδ T cells) are further characterized in that they express at least one or at least a combination of two or more, preferably all, of the following surface markers CD27, CD73, CD69, NKp44, NKp30, and NKG2D, see Fig. 8.

A **sixth** aspect of the invention refers to a cell composition comprising the first subpopulation of cells as defined in the fifth aspect. Thus, the cell composition according to the sixth aspect comprises Vδ1+ γδ T cells characterized in that they express at least the CDla surface marker and, preferably, they do not express at least one, preferably all, of the following surface markers: CD25, CD27, NKp44, NKp30, and NKG2D. A **seventh** aspect of the invention refers to a cell composition comprising the second subpopulation of cells as defined in the fifth aspect. Thus, the cell composition according to the fifth aspect comprises Vδ1+ γδ T cells characterized in that they do not express at least the CDla surface marker and, preferably, they express at least one or at least a combination of two or more, preferably all, of the following surface markers CD27, CD73, CD69, NKp44, NKp30, and NKG2D.

In a preferred embodiment, the population of cells of the sixth and seventh aspect is a substantially pure or homogenous population. In the context of the present invention, a substantially pure population is a population wherein the cells may be substantially isolated cells. In one embodiment, the cell or cell population represent at least about 75%, at least about 80%, at least about 85%, at least about 90%, at least about 95%, at least about 98%, at least about 99% or 100% of the cells in the composition.

An **eighth aspect** of the invention relates to a method of activating and inducing the proliferation of the Vδ1+ γδ T cells defined in the second, third, fourth, fifth, sixth and seventh aspects of the invention. The method may preferably comprise the use of an activating agent such as a yδTCR agonist, including, but not limited to anti-CD3 mAb, and cytokines including, but not limited to, IL-4, IFNγ and IL-15. To this end and as shown in example 1 or 2, a cell composition comprising Vδ1+ γδ T cells obtained or obtainable according to the first aspect of the invention are preferably depleted of αβ T cells by any useful technique such as magnetic cell sorting using anti-TCRαβ mAbs and magnetic beads (Miltenyi Biotec). TCRαβ-depleted cell suspensions will be cultured, for example for 7 days (2.5 x 10⁵ cells/ml) in RPMI1640 medium, in the presence of an activating agent such as anti-CD3 mAb OKT3 plus cytokines such as IL-2, IL-4, IFN-y, IL-21, IL-15 and/or IL-1β. Cells are then optionally washed and cultured again one or more times, with an activating agent and one or more cytokines. Cultures will be normally stopped by day 10-30, preferably 10-25, more preferably 15-25, more preferably 15-16, or cells may be optionally diluted and subjected to a second round of expansion in the presence of an activating agent and one or more cytokines. It is noted that the combination of the first and third aspect of the invention constitutes a two-step method which is expected to allow for an upgraded CD1a- Vδ1+ T-cell yield. In particular, the method already described in the prior art (Almeida et al., Clin. Cancer Res. 2016) allows for a 2000-fold expansion of Vδ1T cells from peripheral blood in 3 weeks.

It is noted that the final subset of Vδ1+ γδ T cells obtained from the eighth aspect of the invention shall stably express natural functional cytotoxicity receptors associated with enhanced cytotoxicity against lymphoid leukemia cells.

A merely illustrative further potentially useful method of activating and inducing proliferation of the Vδ1+ γδ T cells defined in the second, third, fourth, fifth, sixth and seventh aspects of the invention , would be by cultivating these cells in an adequate culture medium in the presence of yδTCR agonists, preferably by regular addition (more preferably continuous addition) of said agonists, preferably soluble or immobilized, and in the presence of at least one cytokine such as those selected from the list consisting of IL-2, IL-4, IL-7, IL-9, IFN-y, IL-21, IL-15 and/or IL-1β, preferably by regular addition (more preferably continuous addition) of said cytokine or cytokines. The said yδTCR agonists and cytokines are added to said culture of cells at the time of the culturing and also throughout the culturing period, preferably every 3-6 days, so that the concentration of yδTCR agonists and cytokines in the said culture is always typically more than zero. The addition of said yδTCR agonists and cytokines could be carried out until at least 40% of the cells express natural cytotoxicity receptors, more preferably at least 50%, 60%, 70%, 75%, 80%, 85%, 95%. In a more preferred embodiment, the addition of said yδTCR agonists and cytokines could be carried out until it is achieved more than 50 million, more than 100 million, more than 200 million of viable and functional cells that express natural cytotoxicity receptors, namely the natural cytotoxicity receptors comprise or consist of NKp30. Preferably, the addition of said yδTCR agonists and cytokines could be carried out until at least 40% of the cells express NKp30, more preferably at least 50%, 60%, 70%, 75%, 80%, 85%, 95%, 100%. In a preferred embodiment of the disclosed method the said cytokine means common cytokines, preferably interleukin, namely IL-2, IL-4, IL-7, IL-9, IL-12, IL-15, IL-21, IFN-y, IL-1β or mixtures thereof, among others, preferably IL-7. The interleukin used may be of human or animal origin, preferably of human origin. It may be a wild-type protein or any biologically active fragment or variant, that is, to say, capable of binding its receptor and inducing activation of γδ T cells in the conditions of the method according to the invention. More preferably, the cytokines may be in soluble form, fusioned or complexed with another molecule, such as for example a peptide, polypeptide or biologically active protein. Preferably, a human recombinant cytokine is used. More preferably, the range of interleukin concentration could vary between 1-10000 U/ml, even more preferably between 100-1000 U/ml. In other preferred embodiment of the disclosed method the regular addition of said yδTCR agonists and yc-cytokines could be performed for 2-60 days, more preferably between 9-25 days, even more preferably between 15-25 days, namely 15, 16, 17, 18, 19, 20 or 21 days.

A **ninth aspect** of the invention refers to a composition comprising the activated and expanded population of Vδ1+ γδ T cells obtained or obtainable according to the eighth aspect of the invention. Preferably, the invention refers to a composition comprising an allogenic activated and expanded population of Vδ1+ γδ T cells obtained or obtainable according to the eighth aspect of the invention.

In a preferred embodiment of the invention, the compositions of the second to seventh or ninth aspects is used to produce chimeric antigen receptor (CAR) T cells.

A **tenth** aspect of the invention refers to a composition comprising CAR T cells obtained or obtainable with any of the compositions of the second to seventh or ninth aspects of the invention.

In a preferred embodiment of the invention, the compositions of the second to seventh or ninth aspects are an injectable. In a preferred embodiment, the injectable composition comprises a cell population composed of more than 80%, namely more than 80%, 85%, 90%, 95%, of functional Vδ1+ γδ cells of the invention expressing functional natural cytotoxicity receptors, wherein it, preferably, comprises more than 100 million of Vδ1+ γδ cells of the invention expressing functional natural cytotoxicity receptors. Preferably the composition also comprises a pharmaceutically acceptable agent or carrier and, more preferably, a stabilizing agent, namely as human serum albumin. The cells may be autologous, that is to say, derived from a same biological preparation (or from a same donor), however, the cells are more preferably of allogenic nature, that is to say, not derived from a same biological preparation (or from a same donor). More preferably, they are obtained by a method such as method described by disclosed subject matter. Another aspect of the disclosed subject matter is the use in medicine of composition that comprises the cells of the second, fourth or fifth aspect of the invention.

In a more preferred embodiment, the compositions disclosed in the second to seventh or ninth aspects could be used in autologous or allogeneic adoptive cell therapy, tumor or cancer treatment, tumor or cancer immunotherapy, and/or leukemia treatment, or for treatment of acute lymphoblastic leukemia, acute myelogenous leukemia, chronic myelogenous leukemia, chronic lymphocytic leukemia, multiple myeloma, Burkitt's lymphoma, follicular lymphoma, T-cell lymphoma, breast carcinoma, lung carcinoma, prostate carcinoma, colon carcinoma, bladder carcinoma, renal cell carcinoma, or skin melanoma, among others. More preferably, the composition disclosed in the fifth aspect could be used in autologous or allogeneic adoptive cell therapy, tumor or cancer treatment, tumor or cancer immunotherapy, and/or leukemia treatment, or for treatment of acute lymphoblastic leukemia, acute myelogenous leukemia, chronic myelogenous leukemia, chronic lymphocytic leukemia, multiple myeloma, Burkitt's lymphoma, follicular lymphoma, breast carcinoma, lung carcinoma, prostate carcinoma, colon carcinoma, bladder carcinoma, renal cell carcinoma, or skin melanoma, among others.

In a more preferred embodiment, the compositions disclosed in the present invention could be used in the treatment of a viral infection.

The present invention further includes the following clauses:
1. *In vitro* use of Notch ligands to generate *de novo* Notch-induced differentiated Vδ1+ γδ T cells, preferably allogenic Notch-induced differentiated Vδ1+ γδ T cells, from a cell population comprising human HPCs, such as CD34+ cord blood HPCs, and/or CD34+ ETPs, wherein the Notch ligand is Jag2 (Jagged 2).
2. An i*n vitro* method to generate *de novo* Notch-induced differentiated Vδ1+ γδ T cells, preferably allogenic Notch-induced differentiated Vδ1+ γδ T cells, from a cell population comprising human HPCs such as CD34+ cord blood HPCs, and/or human ETPs, the method comprising:
   *a*. cultivating the cell population comprising the HPCs and/or ETPs in an adequate culture medium comprising a Jag2 Notch ligand or a Notch receptor agonist, preferably immobilized on a substrate or on a cell line; and
   *b.* maintaining the cells in culture for a duration of time sufficient to produce the Vδ1+ γδ T cells, preferably the duration of time is between about 2 and about 15 weeks, and the said Notch ligand or agonist should be present or added in an adequate amount to said culture of cells at the time of the culturing and also throughout the culturing period,
3. The method according to any of clauses 1 or 2, wherein the human HPCs are derived from CD34+ cord blood HPCs, CD34+ bone marrow HPCs, CD34+ peripheral blood HPCs, or from CD34+ cells derived from pluripotent stem cells such as iPSCs.
4. The method according to any of clauses 1 or 3, wherein the human hematopoietic progenitor cells are human CD34+ ETPs.
5. A cell composition comprising the human cytotoxic Vδ1+γδ cells obtained or obtainable according to any of clauses 1 to 4.
6. A method of activating and inducing proliferation of the Vδ1+ γδ T cells obtained by the method of any of clauses 1 to 5, the method comprising:
   a. cultivating a cell composition comprising the Vδ1+ γδ T cells obtained by the method of any of clauses 2 to 4 in an adequate culture medium in the presence of yδTCR agonists, preferably by regular addition (more preferably continuous addition) of said agonists, preferably soluble or immobilized, and in the presence of at least one cytokine such as those selected from the list consisting of IL-2, IL-4, IL-7, IL-9, IFN-y, IL-21, IL-15 and/or IL-1β; and
   b. adding said yδTCR agonists and cytokines until at least 40% of the cells express natural cytotoxicity receptors, more preferably at least 50%, 60%, 70%, 75%, 80%, 85%, 95% of the cells express natural cytotoxicity receptors.
7. A cell composition comprising the human cytotoxic Vδ1+γδ cells obtained or obtainable according to clause 6.
8. The composition of clause 7, wherein the composition comprises a cell population composed of more than 80%, functional Vδ1+ γδ cells expressing functional natural cytotoxicity receptors, wherein it, preferably, comprises more than 100 million of Vδ1+ γδ cells expressing functional natural cytotoxicity receptors.
9. The composition of any of clauses 7 or 8, wherein the composition is a pharmaceutical composition that also comprises a pharmaceutically acceptable agent or carrier and, more preferably, a stabilizing agent, namely as human serum albumin.
10. The composition of any of clauses 7 to 9, wherein the composition is for use in allogenic adoptive cell therapy, tumor or cancer treatment, tumor or cancer immunotherapy, and/or leukemia treatment, or for treatment of acute lymphoblastic leukemia, acute myelogenous leukemia, chronic myelogenous leukemia, chronic lymphocytic leukemia, multiple myeloma, Burkitt's lymphoma, follicular lymphoma, t-cell Lymphoma, breast carcinoma, lung carcinoma, prostate carcinoma, colon carcinoma, bladder carcinoma, renal cell carcinoma, or skin melanoma.
11. The composition of any of clauses 7 to 9, wherein the composition is for use in autologous adoptive cell therapy, tumor or cancer treatment, tumor or cancer immunotherapy, and/or leukemia treatment, or for treatment of acute lymphoblastic leukemia, acute myelogenous leukemia, chronic myelogenous leukemia, chronic lymphocytic leukemia, multiple myeloma, Burkitt's lymphoma, follicular lymphoma, breast carcinoma, lung carcinoma, prostate carcinoma, colon carcinoma, bladder carcinoma, renal cell carcinoma, or skin melanoma.
12. Use of the composition of any of clauses 7 or 8 for the manufacture of CAR T cells.
13. A CAR T cell composition comprising the human cytotoxic Vδ1+ γδ cells obtained or obtainable according to clause 12.
14. The composition of clause 13, wherein the composition is a pharmaceutical composition that also comprises a pharmaceutically acceptable agent or carrier and, more preferably, a stabilizing agent, namely as human serum albumin.
15. The composition of any of clauses 13 to 14, wherein the composition is for use in allogenic adoptive cell therapy, tumor or cancer treatment, tumor or cancer immunotherapy, and/or leukemia treatment, or for treatment of acute lymphoblastic leukemia, acute myelogenous leukemia, chronic myelogenous leukemia, chronic lymphocytic leukemia, multiple myeloma, Burkitt's lymphoma, follicular lymphoma, T-cell lymphoma, breast carcinoma, lung carcinoma, prostate carcinoma, colon carcinoma, bladder carcinoma, renal cell carcinoma, or skin melanoma.
16. The composition of any of clauses 13 to 14, wherein the composition is for use in autologous adoptive cell therapy, tumor or cancer treatment, tumor or cancer immunotherapy, and/or leukemia treatment, or for treatment of acute lymphoblastic leukemia, acute myelogenous leukemia, chronic myelogenous leukemia, chronic lymphocytic leukemia, multiple myeloma, Burkitt's lymphoma, follicular lymphoma, T-cell lymphoma, breast carcinoma, lung carcinoma, prostate carcinoma, colon carcinoma, bladder carcinoma, renal cell carcinoma, or skin melanoma.

The following examples merely serve to illustrate the present invention but do not limit the same.

### Examples

### Example 1. Generation of V61+ human T cells from human CD34+ early thymic progenitors (ETPs).

- *In vitro* culture conditions for *de novo* generation of Vδ1+ γδ T cells from human CD34+ ETPs activated with Jag2 (STEP1).

Human postnatal thymocytes were isolated by mechanical disruption and Ficoll-Hypaque (Lymphoprep^{™}; ATOM) centrifugation of thymic tissue removed during corrective cardiac surgery of patients aged 3 days to 4 years, in accordance with the Declaration of Helsinki and after informed consent was provided. Experiments were performed in accordance with approved guidelines established by the Research Ethics Board of the Spanish Research Council (CSIC). CD34+ ETPs were isolated from thymocyte cell suspensions by CD34-magnetic cell sorting (Dynal, CD34 Progenitor Cell selection System, Invitrogen) and further depletion of CD34+CD1a+ and CD34+CD123+ progenitors using anti-CDla and anti-CD123 MicroBeads (AutoMACS, Miltenyi Biotec). Isolated CD34+ ETPs (>96% CD34+CD1a-CD123-) were cultured (10⁵ cells/well) in p24 well plates seeded with OP9 stromal cells which were transduced with GFP as cell tracer and either DLL1, DLL4, Jag1 or Jag2 Notch ligands, which were shown to be expressed at similar surface levels, or with GFP alone as control. The transduction of OP9 cells with plasmids encoding for GFP, DLL1, DLL4, Jag1 or Jag2 results in an overexpression of said proteins on the cells, particularly on their surface in the case of the Notch Ligands. In particular, the increased expression of Jag2 in transduced OP9 cells was of about 500-fold increased expression as measured by flow cytometry (Fig. 10). Cultures were set up in α-MEM medium (Gibco) supplemented with 20% fetal calf serum (FCS), 2 mmol/l of L-glutamine, 200 IU/ml of recombinant human (rh) IL-7 (NIBSC) and 100 IU/ml of rhFlt3L (PeproTech). Cultures were re-seeded and analysed by flow cytometry for the generation of γδ T cells every 3-4 days for up to 30 days. These analyses showed that Jag2 is the most efficient Notch ligand promoting the differentiation of γδ T cells (Figure 1), while no cell differentiation was induced when OP9 cells expressed only GFP (Fig 7). In fact, Jag2-mediated signalling results in a preferential γδ over αβ T-cell production with a 300-fold versus a 20-fold yield, respectively, over 30 days. Remarkably, flow cytometry analyses performed with anti-Vδ1 and anti-Vδ2 mAbs have revealed that γδ T cells differentiated from ETPs in response to human Jag2 signalling are preferentially Vδ1+ cells and produce IFNγ but not IL-17 (Figure 2). Therefore, up to 100 Vδ1+ γδ T cells displaying the features of antitumor peripheral γδ T cells can be generated from a single human ETP activated with Jag2 (Figure 3).

### Example 2. Generation of V61+ human T cells from umbilical cord blood CD34+ hematopoietic progenitor cells (HPCs).

- *In vitro* culture conditions for *de novo* generation of Vδ1+ γδ T cells **from human cord blood CD34+ HPCs** activated with Jag2 (STEP1).

Umbilical Cord Blood samples were obtained from the Centro de Transfusion de la Comunidad de Madrid, in accordance with approved guidelines established by the Research Ethics Board of the CSIC. HPCs were obtained from Ficoll Hypaque-isolated cell samples by immunomagnetic sorting using the CD34 Progenitor Cell Isolation Kit (Miltenyi Biotec). Sorted populations were proved >98% CD34+ and negative for CD3, CD4, CD8, CD13, CD14, CD19, and CD56 lineage markers (Lin-) on reanalysis. Isolated cord blood CD34+ HPCs were cultured (10⁵ cells/well) in p24 well plates seeded with OP9 stromal cells expressing human Jag2 Notch ligands, in α-MEM medium (Gibco) supplemented with 20% fetal calf serum (FCS), 2 mmol/l of L-glutamine, and 200 IU/ml of recombinant human (rh) IL-7 (NIBSC), 100 IU/ml of rhFlt3L (PeproTech) and 100 IU/ml rhSCF (PeproTech). Generation of differentiated γδ naïve T cells was analysed every 3-4 days for up to 9 weeks, revealing a 4000-fold total cell expansion and a 210-250 γδ T-cell yield (Figure 4). Up to 40% of these Jag2-diferentiated γδ naïve T cells were Vδ1+ (Figure 4). Therefore, up to 100 million of Vδ1+ γδ T cells can be generated from one million of human cord blood HPC activated with Jag2 (Figure 5).

### Example 3. Generation of non-activated naive V61+ human T cells from umbilical cord blood CD34+ hematopoietic progenitor cells (HPCs) distinct from V61+ γδ T cells present in the adult peripheral blood.

Vδ1+ γδ T cells generated from CD34+ HPCs isolated from cord blood and cultured with OP9 cells overexpressing Jag2 represent a heterogeneous cell population that includes phenotypically immature CD1a+ and mature CD1a- Vδ1+ cells. The mature CD1a- Vδ1+ γδ T cell subset is mostly composed of non-activated naïve CD25- CD27+ cells (Figure 8) that, in contrast to Vδ1+ γδ T cells isolated from adult peripheral blood express the cytotoxic NK receptors NKp30 and NKG2D (Figure 9).

The following Table 1 and Figure 6 show the comparative cell yields and phenotypes of Vδ1+ γδ T cells generated from human CD34+ CB HPCs receiving human Jag2 signaling (CB-Jag2), or *ex vivo*-isolated from CB (CB ex vivo) or peripheral blood (PB ex vivo). Mean ± SD percentages of TCRγδ+ cells and Vδ1+ cells within T cells recovered from CB-Jag2 cells derived from a single CB unit (n=4), or from CB ex vivo (n=4), or PB ex vivo (n=3) cells isolated from single CB or PB units, respectively.

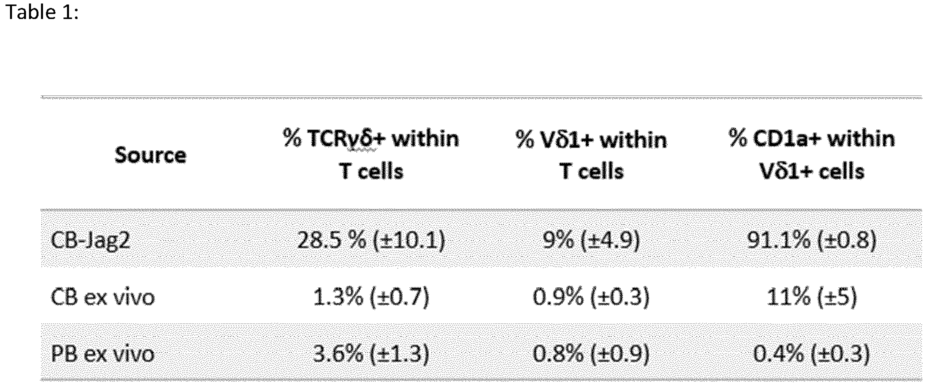

Further, the following Table 2 shows the comparative cell yields in absolute numbers of Vδ1+ γδ T cells generated from human CD34+ CB HPCs receiving human Jag2 signaling (CB-Jag2), or *ex vivo*-isolated from CB (CB ex vivo) or peripheral blood (PB ex vivo) per bag (unit) of blood.

| | **Gated on total cells (%±SEM)** | | | **Cell Nº from CB or PB unit** |
|---|---|---|---|---|
| Source | TCRαβ+ | TCRγδ | Vδ1+ | Vδ1+ (x10⁶±SEM) |
| CB-Jag2 (day 50-60) | 2 ± 0,61 | 7.1 ± 1,12 | 2.4 ± 0.42 | 71,75 ± 42.3 |
| PB ex vivo | 75.85 ±4.25 | 2.7 ±0.71 | 0.51 ± 0.39 | 1.53 ± 0.8 |

Every bag or unit of CB comprises approximately a million of CD34+ progenitor cells, from which an average of 71.75 million of Vδ1+ γδ T cells are produced. On the other hand, every bag or unit of PB comprises an average of 300 million of total cells, from which 0.51% on average are Vδ1+ γδ T cells that represent 1.53 million of Vδ1+ γδ T cells. Thus, the method for producing Vδ1+ γδ T cells from cord blood (CB) and OP9 overexpressing Jag2 is highly efficient, producing an average of 46 times more Vδ1+ γδ T cells from cord blood precursors than from peripheral blood (PB) cells.

Importantly, the Vδ1+ γδ T cells isolated from PB are mainly CD1a- (see Table 1 and Fig. 6), while only 0.4% of cells were found to be CD1a+ from PB ex vivo. However, as shown in Table 1 and Fig. 6, about 9-10% of the cells produced from CB cocultured with OP9-Jag2 cells are CD1a-. This is also observed in the following representative experiment (from a total of 4 experiments), where the percentages of CD1a+ and CD1a- from the Vδ1+ γδ T cells obtained from a unit of CB were measured:

| | **Starting 1x10⁶ CB HPCs** | |
|---|---|---|
| | Vδ1+T cells | Vδ1 CD1a- |
| **EXP.2** | 70x10⁶ | 7x10⁶ |

In view of the above, it can be concluded that from a bag of PB, about 1.53 millions of CD1a- Vδ1+ γδ T cells are obtained, while from a bag of CB and following the method claimed herein, 7 million of CD1a- and 63 million of CD1a+ Vδ1+ γδ T cells are obtained on average.
- TCR-dependent activation and expansion of generated Vδ1+ γδ naive T cells as reported for DOTs (STEP2).

Vδ1+ naive γδ T cells generated *de novo* as described above from cord blood HSCs, in a TCR-independent Jag2-Notch-dependent manner (STEP1), will be activated and expanded *in vitro,* as shown for peripheral blood Vδ1+ T cells (Almeida et al., Clin. Cancer Res. 2016) using anti-CD3 mAbs and cytokines (STEP2), following the protocol named herein STEP 2. To this end, cells differentiated from cord blood CD34+ HSCs (STEP1), will be depleted of αβ T cells by magnetic cell sorting using anti-TCRαβ mAbs and magnetic beads (Miltenyi Biotec). TCRαβ-depleted cell suspensions will be cultured for 7 days (2.5 x 10⁵ cells/ml) in serum-free culture medium (OpTimizer-CTS), supplemented with autologous plasma (i.e. 5% autologous plasma) or human AB serum and 2 mmol/l of L-glutamine in the presence of 1 µg/ml of anti-CD3 mAb OKT3 plus 100 ng/ml rh IL-4, 70 ng/ml IFN-γ, 7 ng/ml IL-21, and 15 ng/ml IL-1β (Peprotech). Cells will be washed at day 7 and cultured for 4 additional days with 2 µg/ml OKT3 plus 13 ng/ml IL-21 and 70 ng/ml IL-15. By day 11, cells will be washed, 1/6 diluted, and cultured for 4-5 additional days in the presence of 2 µg/ml OKT3 plus 100 ng/ml IL-15. Cultures will be stopped by day 15-16, or cells will be 1/3 diluted and subjected to a second round of expansion until day 18-21 in the presence of 1 µg/ml OKT3 plus 70 ng/ml IL-15 and 30 ng/ml IFN-γ. This two-step method is expected to allow for an improved CD1a- Vδ1+ T-cell yield in comparison with state of the art methods.

## Claims

1. *In vitro* use of the Jag2 Notch ligand to generate a cell composition that comprises a higher amount of γδT cells than αβ T cells from a cell population comprising human HPCs, such as CD34+ cord blood HPCs, and/or CD34+ ETPs, wherein the Jag2 Notch ligand is expressed on the surface of a stromal cell line, and wherein said stromal cell line presents a statistically significant increased expression of the Jag2 Notch ligand as compared to a reference stromal cell, wherein said reference stromal cell is a stromal cell expressing basal levels of said ligand.

2. The use according to claim 1, wherein the stromal cell line is OP9 cell line.

3. An *in vitro* method to generate a cell composition that comprises a higher amount of γδ T cells than αβ T cells, preferably allogenic Notch-induced differentiated γδ T cells, from a cell population comprising human HPCs such as CD34+ cord blood HPCs, and/or human ETPs, the method comprising:
a. cultivating the cell population comprising the HPCs and/or ETPs in an adequate culture medium comprising a cell population of a stromal cell line, wherein said stromal cell line presents a statistically significant increased expression of the Jag2 Notch ligand as compared to a reference stromal cell, wherein said reference stromal cell is a stromal cell expressing basal levels of said ligand; and
*b.* maintaining the cells in culture for a duration of time sufficient to produce the γδ T cells, preferably the duration of time is between about 2 and about 15 weeks, and the said Notch ligand or agonist should be present or added in an adequate amount to said culture of cells at the time of the culturing and also throughout the culturing period.

4. The method according to claim 3, wherein the human HPCs are derived from CD34+ cord blood HPCs, CD34+ bone marrow HPCs, CD34+ peripheral blood HPCs, or from CD34+ cells derived from pluripotent stem cells such as iPSCs.

5. The method according to any of claims 3 to 4, wherein the human hematopoietic progenitor cells are human CD34+ ETPs.

6. The method according to any of claims 3 to 5, wherein at least 30% of the γδ T cells are Vδ1+ γδ T cells.

7. A cell composition comprising a higher amount of γδT cells than αβ T cells, obtained or obtainable according to any of claims 3 to 6.

8. The cell composition according to claim 7, wherein the cell composition is **characterized in that** at least 30% of the γδ T cells are Vδ1+ γδ T cells.

9. The cell composition according to claim 8, **characterized in that** the population of Vδ1+ γδ T in turn comprises:
a. a first cell population **characterized in that** they express the immature surface cell marker CDla (CD1a+ Vδ1+ γδ T cells), and
b. a second cell population **characterized in that** they do not express the immature surface cell marker CDla (CD1a- Vδ1+ γδ T cells).

10. The cell composition according to claim 8, wherein the first cell population is **characterized in that** the cells do not express the surface cell markers, CD25, CD27, NKp44, NKp30, and NKG2D.

11. The cell composition according to claims 9 or 10 wherein the second cell population is **characterized in that** the cells express at least one or at least a combination of two or more, preferably all, of the surface markers CD27, CD73, CD69, NKp44, NKp30, and NKG2D.

12. The cell composition according to any of claims 7 to 11, wherein the first cell population represents at least 90% of the total Vδ1+γδ T cells comprised in the cell composition.

13. A method of activating and inducing proliferation of the Vδ1+γδ T cells obtained by the method of any of claims 3 to 6, the method comprising:
a. cultivating a cell composition comprising the Vδ1+ γδ T cells obtained by the method of any of claims 3 to 6 in an adequate culture medium in the presence of yδTCR agonists, preferably by regular addition (more preferably continuous addition) of said agonists, preferably soluble or immobilized, and in the presence of at least one cytokine such as those selected from the list consisting of IL-2, IL-4, IL-7, IL-9, IFN-y, IL-21, IL-15 and/or IL-1β; and
b. adding said yδTCR agonists and cytokines until at least 40% of the cells express natural cytotoxicity receptors, more preferably at least 50%, 60%, 70%, 75%, 80%, 85%, 95% of the cells express natural cytotoxicity receptors.
